(19) **Europäisches Patentamt**

**European Patent Office**

**Office européen des brevets**

(11) Publication number: **0 253 644 B1**

(12) **EUROPEAN PATENT SPECIFICATION**

(45) Date of publication of patent specification: **01.09.93**   (51) Int. Cl.⁵: **G01N 3/08**, G01N 3/20, G01N 33/34

(21) Application number: **87306253.3**

(22) Date of filing: **15.07.87**

(54) **Sensor for determination of the strength of sheet materials.**

(30) Priority: **18.07.86 US 887292**
    **16.10.86 US 920107**

(43) Date of publication of application:
    **20.01.88 Bulletin 88/03**

(45) Publication of the grant of the patent:
    **01.09.93 Bulletin 93/35**

(84) Designated Contracting States:
    **DE FR GB IT SE**

(56) References cited:
    **EP-A- 0 200 650**
    **CH-A- 475 609**
    **DE-C- 751 614**
    **US-A- 2 966 792**

    **JOURNAL OF PHYSICS E SCIENTIFIC IN-STRUMENTS, vol. 4, no. 8, August 1971, pages 615-618, London, GB; J.L. MARTIN et al.: "A high temperature four-point bending machine for testing thin sheets of refractory materials"**

(73) Proprietor: **MEASUREX CORPORATION**
    **One Results Way**
    **Cupertino, California 95014(US)**

(72) Inventor: **Houghton, Paul Joseph**
    **21050 Bear Creek Road**
    **Los Gatos, CA(US)**
    Inventor: **Anderson, Leonard M.**
    **1355 Stockbridge Drive**
    **San Jose, CA(US)**
    Inventor: **Goss, John Douglas**
    **6151 Hancock Avenue**
    **San Jose, CA(US)**

(74) Representative: **Atkinson, Peter Birch et al**
    **Marks & Clerk Suite 301 Sunlight House**
    **Ouay Street**
    **Manchester M3 3JY (GB)**

## Description

Among the critical characteristics of paper and other sheet materials which are important to both manufacturers and users is strength. Many different methods for measuring strength have been proposed in the past, but virtually all suffer from a great disadvantage, namely, the tests are destructive and cannot be used "on-line". A number of standardized tests have been devised to provide a basis for specifications by whch paper can be bought and sold, and these tests provide arbitrary but nevertheless useful strength indices for comparing the strengths of various papers. Unfortunately, all are destructive, and none can be used "on-line".

Two problems which make the measurement of strength "on-line" difficult arise from the facts that the strength of paper varies across the width of the sheet being produced, and is also different in the machine direction and the cross sheet direction. Since papermaking is a high speed continuous process, large amounts of paper can easily be produced before strength can be confirmed by a later measurement.

Strength specifications for paper are usually given in terms of an empirical destructive test, the more common of these being a standardized tensile test, the so-called "STFI" compression test, and the "burst pressure" or "Mullen" test.

In the standard tensile test, a strip of paper is held betwen two clamps and loaded in tension at a predetermined rate. The loading at failure is taken to be a measure of the tensile strength of the paper. There are a number of standardized procedures which have been adopted to perform this test, e.g., TAPPI Standard T404os-76 and ASTM Standard D828.

The "STFI" compression test for heavy papers is a standardized test whose procedure has been established by the Swedish Technical Forest Institute, as specified by the identifiers: Scan P46 Column 83. In this test a strip of the paper to be tested is held between a pair of clamps which are moved together at a fixed rate while the compressive force is monitored. "Rupture" occurs when the compressive force passes a peak and begins to drop. The force at "rupture" is taken as the compressive strength of the paper. Other standard specifications for this test are, e.g., TAPPI 7818os-76 and ASTM D1164.

The strengths of papers as measured by the foregoing tests typically have different values depending on whether the test strip is cut in the machine direction or the cross direction.

A "Mullen" or burst pressure test is conducted by clamping a sample of the paper between two circular clamping rings having a specified standard inside diameter, and building up pressure on one side of the paper until the paper bursts (using a rubber diaphram and liquid pressure). The pressure required to burst the paper is known as the "burst pressure" and is the figure often used to specify the required strength. Common burst pressure specifications are TAPPI 403os-76 and ASTM D774.

Needless to say, none of these tests lend themselves to use in connection with the continuous measurement of paper strength. Because of their widespread popularity, however, it is desirable that any method used to measure the strength of paper provides results which correlate with one of the recognized standard tests.

EP-A-0 200 650 (priority 02.05.85; publication 10.12.86) discloses a system and process for continuously measuring the strength of a moving sheet of material, e.g. paper. The system comprises a circular ring above the aperture of which is provided a freely rotatable wheel supported on a bracket which permits vertical movement of the wheel. A load cell is associated with the wheel to provide a measure of the upward force being applied to the wheel. A tachometer is associated with the wheel, and there is further provided a computer for receiving and processing signals from the load cell and tachometer.

In use, a sheet of material travels over the ring in contact therewith. The wheel deflects the travelling sheet a fixed distance into the aperture of the ring. Increases and decreases in the tension and elastic modulus (i.e. the ratio of stress to strain in the sheet) impart variable forces on the load cell. Signals from the load cell are passed to the computer as are signals from the tachometer corresponding to the rotation of the wheel and this velocity of the sheet. The computer utilises the data to determine the strength of the sheet in accordance with equations as set out in EP-A-0 200 650.

EP-A-0 200 650 only proposes the use of a solid ring (i.e. a ring with an unbroken circumference) which does not allow separate determinations of machine and cross direction tensile and compression strengths (which can be different).

The major factors which influence the strength of paper, e.g., its basis weight and thickness, also affect the elastic modulus and bending stiffness of the sheet. We have found that these latter factors can be sensed in a sheet under tension in such a way that the actual strength of a sheet can be determined by computation from the output of the sensor. The preferred form of strength sensor used senses a characteristic of the paper which will be referred to as "elastic modulus" since the characteristic is related to the modulus, but is not actually a measure of the elastic modulus of the sheet itself. Quotation marks are

2

used around the term "elastic modulus" to indicate that the function, while related to the elastic modulus is really an empirically derived factor which depends on other characteristics such as bending stiffness also. In any particular paper making set up changing the speed of the web also affects the strength of the resulting product.

Elastic modulus is difficult, if not impossible to measure directly on a moving web of paper or other sheet material. However, the present application describes a sensor which can sense a physical characteristic of the sheet related to elastic modulus and can provide an output which, when taken together with the other factors such as one or more of the sheet basis weight, thickness, sheet tension and velocity, can be used to determine sheet strength continuously and non-destructively. This sensor can provide a measurement which allows a strength determination to be made which correlates well with the popular "Mullen" test. In the present application, a sensor is described which not only allows a Mullen determination to be made but allows separate determinations of machine and cross direction tensile and compression strengths.

According to the present invention there is provided a sensor for non-destructively sensing a physical characteristic related to the strength of a moving sheet of material e.g. paper, comprising

a) a plurality of contact means for supporting one side of a moving sheet against a deflecting force, said contact means being disposed around an unsupported region into which the sheet may be deflected.

b) deflecting means for non-destructively deflecting said moving sheet into the unsupported region so that the sheet presses against the contact means.

c) force measuring means operatively associated with a first contact means for sensing the force of the sheet against said first contact means and producing an output indicative of the force of the sheet against said first contact means; and

d) computing means operatively associated with the force measuring means and for calculating the physical characteristic of the sheet material based upon at least the output of said force measuring means.

Preferably, there are four of said contact means which together define a ring with each contact means occupying approximately 90 degrees of the ring circle. Each segment is preferably supported on a pair of leaf springs and is associated with a sensing device (such as a load cell) so that the deflecting means (e.g. a free running wheel) acting on the sheet in the center of the ring will deflect the sheet and result in an output from each of the four load cells, with the output level being dependent in part on sheet characteristics. The four segments are preferably aligned so that two are sensitive to machine direction characteristics of the sheet and two are sensitive to cross direction characteristics.

In the preferred embodiment, the computer accepts the outputs of each of the load cells, and may also accept outputs from one or more other sensors which sense basis weight, sheet thickness, velocity, tension and certain correction factors. The computer calculates the sheet strength using these signals, e.g. in accordance with the empirical equations described later.

It should be understood that while references are made to a specific standardized test herein, the reference is intended to be exemplary only and not limiting. The present invention can be utilized to provide determinations which correlate well with a wide variety of standardized tensile, compressive and burst pressure tests. Alternatively, the present invention can be utilized to provide an arbitrary index of strength which will allow comparisons of strength as between products independent of any existing standardized system.

The invention will be further described by way of example only with reference to the accompanying drawings, in which:

Fig. 1 is a perspective view of one embodiment of sensing portion of the invented apparatus as installed in a paper making machine.

Fig. 2 is a partial cross-sectional side view of the sensor for sensing the "elastic modulus" of the paper;

Fig. 3 is a top plan view of the portion of the lower gauge support member in the region of the sensing ring;

Fig. 4 is a fragmentary cross-sectional view of the sensing wheel;

Fig. 5 is a block diagram of one embodiment of the electronic portion of the invention; and

Fig. 6 is a schematic side view of one means for providing a signal responsive to web tension.

Paper and other sheet-like materials are ordinarily made in a continuous sheet by high speed machines, often several hundred meters in length. The process for making paper involves laying a wet mass of wood pulp onto a moving wire fabric belt, drying the mass, and finally calendering the sheet to give it the desired surface finish. The present invention is most advantageously used to monitor the strength of the paper after the final calendering operation, and before the paper is rolled up on the final reel. A windup motor maintains a constant tension in the sheet between the calender and the reroll reel. Since the strength of the paper produced may vary across the sheet as well as along the sheet, the present invention preferably involves

3

the use of a scanning system whereby the sensors scan cross the width of the paper while the paper is being fed out of the calender and into the reroll system. In this way the variations in strength, side to side, may be determined, as well as the strength of the paper at each section along its length.

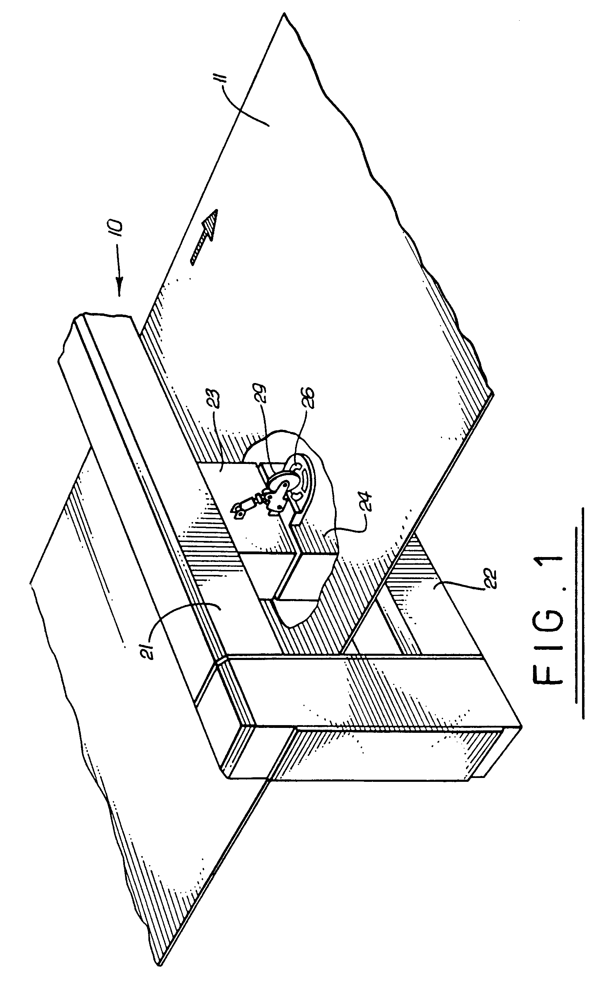

Figure 1 shows a scanning station 10 which, as noted above, is preferably located after the final calender rolls and before the reroll system. A web of paper 11 can be seen passing through the scanning station 10 betwen two transverse beams 21 and 22, on which are mounted upper and lower gauge support members 23 and 24. The web of paper 11 in Figure 1 is shown with a cut out area so that the relationship between the gauge support members can be seen. A motor within the scanning station is coupled to, and drives the gauge support members 23, 24 back and forth across the width of the paper in a continuous scanning motion, keeping them in alignment at all times.

The gauge support members may carry four or more sets of sensors to provide data that can be used to calculate paper strength. Four factors which may be used are basis weight, thickness, "elastic modulus", and the velocity of the web. Means for determining basis weight, thickness, and paper velocity are all known in the prior art and are therefore not separately shown on the drawings or discussed individually herein. Thickness and paper velocity are relatively simple to measure, and many methods are known in the prior art. Basis weight is a more complex matter, but a suitable method is disclosed in U.S. Patent No. 3,757,122 issued to Bossen et al.

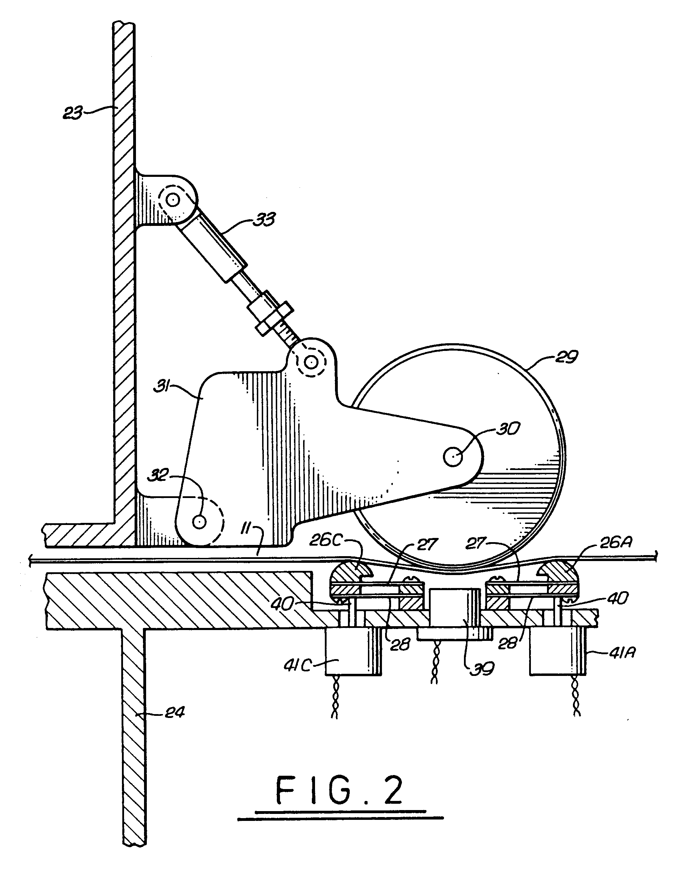

The fourth sensor provides data which relates to the "elastic modulus" of the paper. One such "elastic modulus" sensor used to detect a physical characteristic of the sheet which may be correlated to the sheet or paper strength is shown in Fig. 2. In the particular embodiments of the sensor shown, the characteristic may be sensed by detecting the force required to deflect the sheet a predetermined amount or by detecting the deflection of the sheet when subjected to a predetermined force.

The "elastic modulus" sensor is carried on gauge supports 23, 24. Figure 2 shows a partially sectioned side view of this sensor. Lower gauge support 24 supports a segmented horizontal ring 26A-26D whose top surface is preferably aligned with the paper web 11. While a ring such as ring 16 is the preferred method of support, other forms of contacting structures could be used which contact the paper in the vicinity of a central unsupported region.

Upper gauge support 23 carries a first means such as the pressure wheel assembly which is comprised of bracket 31 and wheel 29. Pin 32 allows the wheel freedom to move up and down while bearings on axle 30 (not shown) permit the wheel to rotate freely. Up and down motion of the wheel 29 is controlled by air cylinder 33. In its extended position, air cylinder 33 positions the lower portion of the periphery of wheel 29 a fixed distance below the top surface of ring 26 and the sheet and the ring 26 are in a cooperative relationship. For purposes of example, and not by way of limitation, if the diameters of wheel 29 and ring 26 are each about 7.5 cm (5 inches), a satisfactory position for the lowest point on wheel 29 may be 0.6 cm (1/4 inch) below the top surface of ring 26. It is also possible to invert the components as shown in Figure 2 so that the ring 26 is above the sheet and the wheel 29 is below the sheet. In a present form of the invention the components are so arranged.

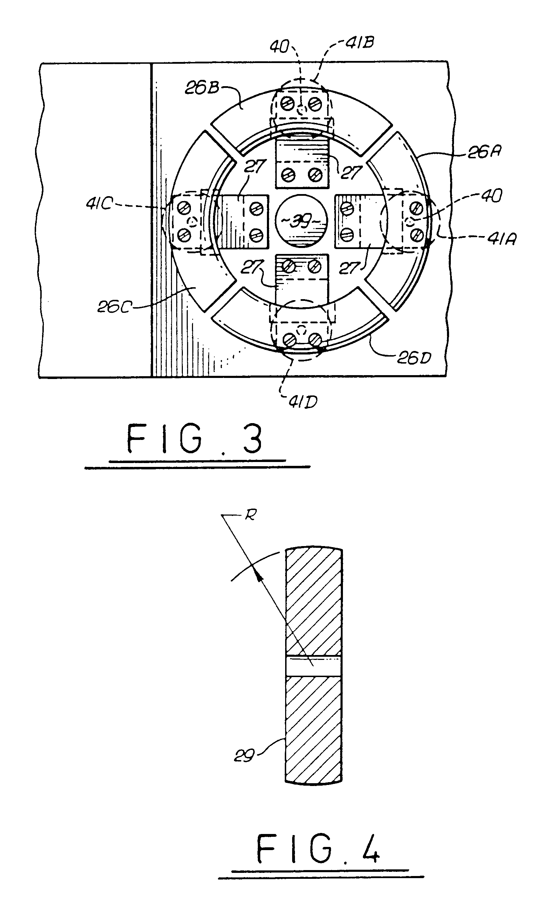

The periphery of wheel 29 is preferably not cylindrical but, rather, is preferably approximately spherical. That is, the radius R as shown in Figure 4 is preferably approximately equal to one-half the wheel diameter. Retraction of air cylinder 33 moves wheel 29 out of the way so that a web of paper 11 can be fed through the scanning station easily upon initial set up. During operation, air cylinder 33 is extended and the sheet is acted upon to move from a normal path to a deflected path in the strength sensor.

It will be understood by those skilled in the art that while a free running wheel for the purpose of deflecting the paper below the top surface of ring 26 is disclosed herein, other structures can be utilized to accomplish the same function.

Figure 3 is a top plan view of the portion of the lower gauge member which supports the segmented ring 26. Referring to both Figures 2 and 3, it can be seen that the ring 26 is comprised of four segments 26A-26D which are each supported by a pair of spaced leaf springs 27 and 28. The leaf springs 27, 28 constrain the segments 26A-26D to move with straight line vertical motion. Linkage pins 40 couple each segment to load cells 41A-41D which provide outputs that are a function of the paper force on the ring segments. The force exerted on each of the segments 26 is a function of several factors including the tension on the sheet, the elastic modulus of the paper, the bending stiffness of the paper, and the physical dimensions of the gauging components. The modulus of elasticity and the bending stiffness of the paper may be different in the machine direction as compared to the cross direction, so that the force applied to load cells 41A and 41C is not necessarily the same as the force applied to load cells 41B and 41D. The difference in tension between the machine direction and the cross-direction also results in a difference in force.

4

We have found that there is a correlation between the force applied to the load cells 41A and C and the machine direction strength of the paper and also a correlation between the force applied to load cells 41B and D and the cross-direction strength of the paper.

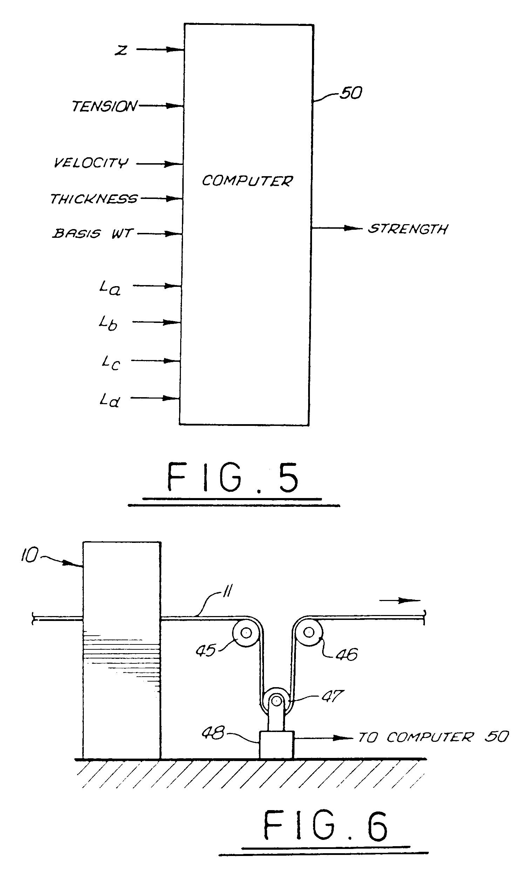

In order to make a strength determination, the outputs of transducers 41A-D are fed to a computer 50 (Fig. 5) which also may receive signals from the other sensors referred to above. The computer 50, by repeated calculations, provides continuous determinations of the strength of the paper as it is being manufactured. Empirical equations have been developed which correlate well with standard machine direction and cross direction strength tests as well as with the non-directional standard burst pressure or "Mullen" test.

The accuracy of the basic equations developed depends upon keeping the alignment of the top surface of ring 26 even with the web of paper 11 as it is being fed into or out of the scanning station 10. If the incoming web 11 is higher or lower than the top surface of ring 26, a correction to the basic equation must be made in order to arrive at an accurate determination of paper strength. We have found that a satisfactory correction can be made by utilizing an additive factor which is a function of the difference between the forces applied to transducers 41A and 41C.

Complete empirical equations for determining directional paper strengths are as follows:

$$S_{md} = A \frac{L_a + L_c}{\overline{L}_a + \overline{L}_c} + (BxW^E xT^F) + CxV^D + Gx(L_a - L_c)^H \quad (1)$$

$$S_{cd} = A \frac{L_b + L_d}{\overline{L}_b + \overline{L}_d} + (BxW^E xT^F) + CxV^D + Gx(L_b - L_d)^H \quad (2)$$

Where

| | |
|---|---|
| $S_{md}$ | is the machine direction strength of the paper; |
| $S_{cd}$ | is the cross direction strength of the paper; |
| A, B, C, D, E, F, G and H | are constants; |
| $L_a$ through $L_d$ | are instantaneous forces applied to transducers 41A through 41D; |
| $\overline{L}_a$ through $\overline{L}_d$ | are the average forces applied to transducers 41A through 41D across the width of the paper; |
| W | is the basis weight of the paper; |
| T | is the thickness of the paper; and |
| V | is the velocity of the paper leaving the calender. |

The above equations have been found to correlate well with both the standard tensile and the standard STFI tests for paper strength, both commonly used. The constants A through H vary depending on which test is being simulated. The last additive factor in each of the above equations represents the correction for web misalignment.

A modification of equations (1) and (2) allows the system to be used to determine the "Mullen" strength of the paper:

$$S_{mu} = A \frac{L_a + L_b + L_c + L_d}{\overline{L}_a + \overline{L}_b + \overline{L}_c + \overline{L}_d} + (BxW^E xT^F) + CxV^D + Gx(L_a - L_c)^H \quad (3)$$

where
$S_{mu}$ is the "Mullen" strength;
All other factors are the same as in equations (1) and (2).

The above equations have been found to be applicable to a wide variety of papers being manufactured. The constants A, B, C, D, E, and F vary somewhat depending on the particular paper being made, and which test is being simulated, but generally fall within the following ranges (where basis weight is in grams per square metre, thickness is in microns, and velocity is in metres per minute:)
A from 20 to 22

B from 0.0008 to 26

C from 0 to 26.6

D from 0.5 to 5

E from 1 to 2

F from -1 to +1

In Imperial units the values of the constants A-F are as set out below (where basis weight is in pounds per 1000 square feet, thickness is in mils, and velocity is in feet per minute:

A from 20 to 22

B from .5 to 5

C from 0 to 0.07

D from 0.5 to 5

E from 1 to 2

F from -1 to +1

The constants G and H must be empirically determined since they are strongly dependent on the particular setup.

It may be noted that the terms of the above equations involving La through Ld are in the form of a ratio with $\bar{L}_a$ through $\bar{L}_d$. This indicates that "elastic modulus" is primarily an indicator of how strength varies across the width of the paper, while the average strength is primarily determined as a function of basis weight, thickness, and web velocity.

Machine rigidity is an important factor in maintaining accuracy of measurement. In particular, it is important that the lowest point on the periphery of wheel 29 be maintained at a constant distance below the top surface or ring 26. If the mechanical rigidity of the structure is such that the spacing cannot be maintained, sensing means can easily be adapted to sense the relative positions of wheel 29 and ring 26, and to apply a correction of the strength equation to account for variations. A conventional sensor for this purpose is shown diagrammatically in Figure 2 identified by the numeral 39. The output of displacement sensor 39, designated as "Z", provides a signal dependent on the deviation in the relative positions of wheel 29 and ring 26 from nominal, and may be used to modify the strength equation. For example, if equation (1) was in use, the first term of the equation would be:

$$f(Z) \quad x \quad A\frac{L_a + L_c}{\bar{L}_a + \bar{L}_c}$$

where f(Z) is an empirical function of the output of sensor 39.

We have also found that by properly combining the outputs of load cells 41A-41D it is possible to make a determination, on line, which accurately correlates with the standard "Mullen" test. In order to make such a determination, the outputs of load cells 41A-41D are fed to a computer 50 along with the output of the displacement sensor 39 (Z). The computer 50 calculates the "Mullen" strength. The presently preferred equation which may be used to determine strength is:

$$S_{mu} = A \left( \frac{L_a + L_c}{(C+Z) \ Te+F)} \right)^H + B \left( \frac{L_b + L_d}{(D+Z) \ Te+G)} \right)^J + E \qquad (4)$$

where $S_{mu}$ is the "Mullen" strength of the paper;

A, B, C, D, E, F, G, H and J    are constants;

$L_a$, $L_b$, $L_c$ and $L_d$    are the output signals from load sensors 41A through 41D, respectively;

Te    is a number representative of the tension in the web; and

Z    is the output of displacement sensor 39.

If the gauge support members are held with adequate rigidity so that there is a sufficiently small relative motion between members 23 and 24, sensor 39 would not be needed, and the terms C + Z and D + Z could be replaced with constants. Similarly, if tension in the web does not vary significantly, the terms Te + F and Te + G could be replaced by constants. Thus, if in a particular installation the variations due to lack of rigidity and variations in tension are low enough, equation (4) could have the following form:

6

$$S_{mu} = A\left(\frac{L_a + L_c}{C}\right)^H + B\left(\frac{L_b + L_d}{D}\right)^J + E \quad (4a)$$

The values of the constants in equations (4) and (4a) will, of course, depend on the particular installation and the circumstances. They are empirically determined, and will, in general, be different as used in equation (4) as compared to (4a). The use of exponents H and J is not intended to indicate a value necessarily different from 1. In some cases, depending on the installation and circumstances, one or both of these exponents may be 1. In addition, the constants may be found to be truly constant (within the desired accuracy of measurement) only if the process variations are relatively small, i.e., within the ranges normally occurring durig the manufacture of a single grade of paper. Unusually large variations in one or more of the process variables may require that one or more of the equation "constants" be adjusted to account for the variation.

There are many ways known in the art for providing a signal to computer 50 which is a function of the tension in the web. One such system is shown schematically in Figure 6. As shown, the web 11 is threaded around three rollers 45, 46 and 47. Rollers 45 and 46 are fixed relative to the paper making machine and roller 47 is restrained from moving in a vertical direction by force transducer 48. The output of force transducer 48 will be a function of the tension of web 11 can be used to provide the quantity Te.

While the above equations provides strength values which correlate with the "Mullen" strength, as noted, it will be evident to those skilled in the art that it is possible to obtain other numbers which are also representative of strength using the strength sensor outputs in connection with different equations, even though such other numbers may not correlate with "Mullen".

For example, if a determination of machine direction and/or cross direction tensile strength is desired, equations in the following forms could be used:

$$S_{md} = A\left(\frac{L_a + L_c}{(C+Z)\ (Te+F)}\right)^H + E \quad (5)$$

and

$$S_{cd} = B\left(\frac{L_b + L_d}{(D+Z)\ (Te+G)}\right)^J + E \quad (6)$$

where

$S_{md}$ and $S_{cd}$ are the machine direction and cross direction tensile strengths, respectively; and the other terms are as previously defined.

By proper choice of the constants, equations (5) and (6) can provide determinations which correlate well with standard tensile tests. While the same letters (A, B, C, etc.) are used in equations 4, 4a, 5 and 6 to represent constants, it should not be assumed that the constants as used in these equations necessarily have the same values. As noted above, the actual values of the constants will depend on the particular installation and circumstances. The comments above concerning modification of equation (4) on account of the effects of rigidity and tension are also applicable to equations (5) and (6) and similar simplified equations can be used if rigidity and/or tension variations are small enough. That is, the terms in the denominator of the first term of the equations could be replaced by a constant.

What has been disclosed is a sensor determining the strength of a sheet of material, such, for example, paper, which is nondestructive and which can be used "on-line" to assure that the paper or other sheet material being manufactured meets the required strength specifications. Various adaptations and modifications of the invention will no doubt occur to those skilled in the art, and such adaptations and modifications within the appended claims are intended to be covered thereby. For example, a presently preferred embodiment of the invention includes a segmented ring with four segments and four load cells, but that is not the only possible number of sheet supporting areas. It is possible to get meaningful information from even a single load cell coupled to one of the segments.

**Claims**

1. A sensor for non-destructively sensing a physical characteristic related to the strength of a moving sheet (11) of material, comprising
    a) a plurality of contact means (26A, 26B, 26C, 26D) for supporting one side of a moving sheet (11) against a deflecting force, said contact means being diposed around an unsupported region into which the sheet may be deflected.
    b) deflecting means (29) for non-destructively deflecting said moving sheet into the unsupported region so that the sheet presses against the contact means.
    c) force measuring means (41A) operatively associated with a first contact means (26A) for sensing the force of the sheet against said first contact means and producing an output indicative of the force of the sheet against said first contact means; and
    d) computing means (50) operatively associated with the force measuring means (41A) and for calculating the physical characteristic of the sheet material based upon at least the output of said force measuring means (41A).

2. A sensor as claimed in claim 1, wherein a plurality of the contact means (26A, 26B, 26C, 26D) are each operatively associated with a respective force measuring means (41A, 41B, 41C, 41D) each of which produces an output for processing by the computer indicative of the force of the sheet against the respective contact means.

3. A sensor as claimed in claim 2 wherein at least one of the contact means (26A) is aligned to sense a physical characteristic in the machine direction and at least one of the second contact means (26B) is aligned to sense a physical characteristic in the cross-direction.

4. A sensor as claimed in any one of claims 1 to 3 wherein the plurality of contact means (26A, 26B, 26C, 26D) comprises a segmented ring.

5. A sensor as in claim 4 wherein each segment (26A, 26B, 26C, 26D) of the ring occupies approximately ninety degrees of said ring.

6. A sensor as claimed in any one of claims 1 to 5, wherein the deflecting means (29) comprises a free-turning wheel pressing against the unsupported region of the sheet 11 on the side thereof opposite the support means (26A, 26B, 26C, 26D).

7. A sensor as claimed in any one of claims 1 to 6, wherein said sheet (11) is paper.

8. A sensor as claimed in any one of claims 1 to 7, wherein the deflecting means (29) is adapted to deflect the sheet a relatively fixed distance.

9. A sensor as claimed in any one of claims 1 to 8, wherein the physical characteristic is failure strength.

10. A sensor as claimed in claim 9, further comprising a deflection sensor (39) for sensing the distance that the sheet (11) is deflected into the unsupported region and for producing a signal indicative of the sensed distance, and wherein the computing means also determines the failure strength based upon the signal.

11. A sensor as claimed in any one of claims 1 to 10, wherein the determined physical characteristic is directionally dependent.

**Patentansprüche**

1. Sensor zum Wahrnehmen auf zerstörungsfreie Weise einer physischen mit der Festigkeit eines sich bewegenden Bogens (11) eines Materials zusammenhängenden Eigenschaft, welcher folgendes umfaßt:

a) eine Mehrzahl an Kontakteinrichtungen (26A, 26B, 26C, 26D) zum Stützen einer Seite eines sich bewegenden Bogens (11) gegen eine ablenkende Kraft, wobei die Kontakteinrichtungen um einen ungestützten Bereich herum angeordnet sind, in den hinein der Bogen abgelenkt werden kann.

b) eine Ablenkungseinrichtung (29) zum Ablenken auf zerstörungsfreie Weise des sich bewegenden Bogens in den ungestützten Bereich hinein, so daß der Bogen gegen die Kontakteinrichtungen drückt.

c) eine betriebsmäßig einer ersten Kontakteinrichtung (26A) zugeordnete Kraftmeßeinrichtung (41A) zum Wahrnehmen der Kraft des Bogens gegen die erste Kontakteinrichtung und zum Erzeugen einer Ausgabe, die die Kraft des Bogens gegen die erste Kontakteinrichtung anzeigt; und

d) eine betriebsmäßig der Kraftmeßeinrichtung (41A) zugeordnete Recheneinrichtung (50) zum Errechnen der physischen Eigenschaft des Bogenmaterials auf der Basis von zumindest der Ausgabe der Kraftmeßeinrichtung (41A).

2. Sensor gemäß Anspruch 1, bei dem mehrere der Kontakteinrichtungen (26A, 26B, 26C, 26D) jeweils betriebsmäßig einer jeweiligen Kraftmeßeinrichtung (41A, 41B, 41C, 41D) zugeordnet sind, die jeweils eine vom Rechner zu verarbeitende Ausgabe erzeugen, die die Kraft des Bogens gegen die jeweilige Kontakteinrichtung anzeigt.

3. Sensor gemäß Anspruch 2, bei dem wenigstens eine der Kontakteinrichtungen (26A) darauf ausgerichtet ist, eine physische Eigenschaft in Maschinenrichtung wahrzunehmen, und wenigstens eine der zweiten Kontakteinrichtungen (26B) darauf ausgerichtet ist, eine physische Eigenschaft in Querrichtung wahrzunehmen.

4. Sensor gemäß einem der Ansprüche 1 bis 3, bei dem die Mehrzahl an Kontakteinrichtungen (26A, 26B, 26C, 26D) einen segmentierten Ring umfaßt.

5. Sensor gemäß Anspruch 4, bei dem jedes Segment (26A, 26B, 26C, 26D) des Rings annähernd neunzig Grad des Rings einnimmt.

6. Sensor gemäß einem der Ansprüche 1 bis 5, bei dem die Ablenkungseinrichtung (29) ein frei drehbares Rad umfaßt, das gegen den ungestützten Bereich des Bogens 11 auf dessen den Stützeinrichtungen (26A, 26B, 26C, 26D) gegenüberliegender Seite drückt.

7. Sensor gemäß einem der Ansprüche 1 bis 6, bei dem es sich beim Bogen (11) um Papier handelt.

8. Sensor gemäß einem der Ansprüche 1 bis 7, bei dem die Ablenkungseinrichtung (29) dafür eingerichtet ist, den Bogen um eine relativ feste Entfernung abzulenken.

9. Sensor gemäß einem der Ansprüche 1 bis 8, bei dem es sich bei der physischen Eigenschaft um die Festigkeitsgrenze handelt.

10. Sensor gemäß Anspruch 9, der ferner einen Ablenkungssensor (39) zum Wahrnehmen der Entfernung, um die der Bogen (11) in den ungestützten Bereich hinein abgelenkt wird, und zum Erzeugen eines Signals, das die wahrgenommene Entfernung anzeigt, umfaßt und bei dem die Recheneinrichtung ebenfalls die Festigkeitsgrenze auf der Basis des Signals bestimmt.

11. Sensor gemäß einem der Ansprüche 1 bis 10, bei dem die bestimmte physische Eigenschaft von der Richtung abhängt.

**Revendications**

1. Capteur destiné à détecter de manière non destructive une caractéristique physique liée à la résistance d'une feuille (11) en déplacement de matière, comprenant

a) une pluralité de moyens de contact (26A, 26B, 26C, 26D) destinés à supporter un côté d'une feuille (11) en déplacement à l'encontre d'une force déflectrice, lesdits moyens de contact étant disposés autour d'une région non supportée dans laquelle la feuille peut être défléchie.

b) un moyen déflecteur (29) destiné à défléchir de manière non destructive ladite feuille en déplacement, dans la région non supportée de sorte que la feuille presse contre les moyens de

9

contact.

c) un moyen de mesure de force (41A) associé activement à un premier moyen de contact (26A), destiné à détecter la force de la feuille contre ledit premier moyen de contact et à produire une sortie indicatrice de la force de la feuille contre ledit premier moyen de contact ; et

d) un moyen de calcul (50) associé activement au moyen de mesure de force (41A) et destiné au calcul de la caractéristique physique de la feuille de matière en se basant sur au moins la sortie dudit moyen de mesure de force (41A).

2.  Capteur selon la revendication 1, dans lequel une pluralité des moyens de contact (26A, 26B, 26C, 26D) sont chacun associés activement à un moyen de mesure de force (41A, 41B, 41C, 41D) respectif dont chacun produit une sortie destinée à être traitée par l'ordinateur et indicatrice de la force de la feuille contre le moyen de contact respectif.

3.  Capteur selon la revendication 2, dans lequel au moins un des moyens de contact (26A) est aligné pour détecter une caractéristique physique dans le sens machine et au moins un des seconds moyens de contact (26B) est aligné pour détecter une caractéristique physique dans le sens travers.

4.  Capteur selon l'une quelconque des revendications 1 à 3, dans lequel la pluralité de moyens de contact (26A, 26B, 26C, 26D) comprend une bague segmentée.

5.  Capteur selon la revendication 4, dans lequel chaque segment (26A, 26B, 26C, 26D) de la bague occupe environ quatre-vingt-dix degrés de ladite bague.

6.  Capteur selon l'une quelconque des revendications 1 à 5, dans lequel le moyen déflecteur (29) comprend une roue tournant librement et pressant contre la région non supportée de la feuille 11 sur le côté de celle-ci opposé au moyen de support (26A, 26B, 26C, 26D).

7.  Capteur selon l'une quelconque des revendications 1 à 6, dans lequel ladite feuille (11) est du papier.

8.  Capteur selon l'une quelconque des revendications 1 à 7, dans lequel le moyen déflecteur (29) est adapté pour défléchir la feuille d'une distance relativement fixe.

9.  Capteur selon l'une quelconque des revendications 1 à 8, dans lequel la caractéristique physique est la résistance à la défaillance.

10. Capteur selon la revendication 9, comprenant en outre un capteur de déflexion (39) destiné à détecter la distance dont la feuille (11) est défléchie dans la région non supportée et destiné à produire un signal indicateur de la distance détectée, et dans lequel le moyen de calcul détermine également la résistance à la défaillance en se basant sur le signal.

11. Capteur selon l'une quelconque des revendications 1 à 10, dans lequel la caractéristique physique déterminée est dépendante du sens.

FIG.1

EP 0 253 644 B1

F I G . 2

FIG. 3

FIG. 4

FIG.5

FIG.6